(19) Europäisches Patentamt
European Patent Office
Office européen des brevets

(11) EP 0 714 284 B1

(12) FASCICULE DE BREVET EUROPEEN

(45) Date de publication et mention de la délivrance du brevet:
**21.03.2001 Bulletin 2001/12**

(21) Numéro de dépôt: **94925512.9**

(22) Date de dépôt: **16.08.1994**

(51) Int Cl.[7]: **A61K 7/48**, A61K 31/70

(86) Numéro de dépôt international:
**PCT/FR94/01008**

(87) Numéro de publication internationale:
**WO 95/05155 (23.02.1995 Gazette 1995/09)**

# (54) UTILISATION D'OLIGOSACCHARIDES DANS LA PREVENTION ET LE TRAITEMENT DU VIEILLISSEMENT DES TISSUS

VERWENDUNG VON OLIGOSACCHARIDEN ZUR VERHÜTUNG UND BEHANDLUNG VON ALTERUNG DER HAUT

USE OF OLIGOSACCHARIDES FOR THE PREVENTION AND TREATMENT OF TISSUE AGEING

(84) Etats contractants désignés:
**AT BE CH DE DK ES FR GB GR IE IT LI LU MC NL PT SE**

(30) Priorité: **17.08.1993 FR 9310054**

(43) Date de publication de la demande:
**05.06.1996 Bulletin 1996/23**

(73) Titulaire: **Johnson & Johnson Consumer France 92130 Issy les Moulineaux (FR)**

(72) Inventeurs:
- **ROBERT, Ladislas**
  **F-94440 Santeny (FR)**
- **ROBERT, Alexandre**
  **F-94440 Santeny (FR)**
- **MOCZAR, Elemer**
  **F-91190 Gif-sur-Yvette (FR)**

(74) Mandataire: **Martin, Jean-Jacques et al**
**Cabinet Régimbeau**
**20, rue de Chazelles**
**75847 Paris cedex 17 (FR)**

(56) Documents cités:

| | |
|---|---|
| EP-A- 0 133 170 | EP-A- 0 504 645 |
| WO-A-93/00067 | FR-A- 2 609 397 |
| US-A- 4 895 838 | US-E- R E28 781 |

- **DATABASE WPI Week 939, Derwent Publications Ltd., London, GB; AN 93-071012 & JP,A,5 017 332 (SHISEIDO) 26 Janvier 1993**
- **DATABASE WPI Week 931, Derwent Publications Ltd., London, GB; AN 93-004724 & JP,A,4 332 795 (LION CORP.) 19 Novembre 1992**
- **DATABASE WPI Week 9049, Derwent Publications Ltd., London, GB; AN 90-366300 & JP,A,2 265 458 (NIKKEN FOOD KK) 30 Octobre 1990**
- **PATENT ABSTRACTS OF JAPAN vol. 9, no. 32 (C-265) (1755) 9 Février 1985 & JP,A,59 176 203 (KANEBO KK)**
- **PATENT ABSTRACTS OF JAPAN vol. 11, no. 167 (C-425) (2614) 28 Mai 1987 & JP,A,62 000 412 (SHISEIDO CO)**
- **PATENT ABSTRACTS OF JAPAN vol. 12, no. 230 (C-508) (3077) 29 Juin 1988 & JP,A,63 023 808 (NOEBIA KK)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention porte sur une composition pour le traitement ou la prévention des manifestations du vieillissement comportant des oligosaccharides ou des dérivés d'oligosaccharides ayant du D-galactose en position terminale non réductrice, ces dérivés agissant en tant que modulateurs de la synthèse et/ou de l'excrétion de l'élastase de fibroblastes.

Il est connu que le tissu conjonctif au niveau du derme résulte de l'activité biosynthétique des fibroblastes qui élaborent la matrice extracellulaire (Labat-Robert J. et al., Elsevier Science Publisher B.V. 248:386-393, 1990).

La matrice extracellulaire est constituée par quatre familles de macromolécules. Il s'agit des collagènes et de l'élastine qui constituent le matériel fibreux du derme, les glycoprotéines de structure qui assurent la cohésion entre les cellules et la matrice extracellulaire et fournissent le matériel microfibrillaire du tissu élastique ; les protéoglycanes qui assurent l'hydratation des tissus et le contrôle du trafic moléculaire.

L'élastine possède un récepteur complexe dans la membrane des cellules fibroblastiques qui contient une sous-unité de 67 kD qui comporte un site de liaison aux hydrates de carbone.

A. Hinek et al. (Science 239:1539-1541, 1988) ont montré que, lorsque la sous-unité 67 kD du récepteur de l'élastine était immobilisée sur une colonne d'affinité dont le ligand était constitué par de l'élastine ou des peptides de l'élastine, le lactose (1 mM) était capable d'éluer cette protéine 67 kD, et que le galactose était également efficace dans le déplacement de la liaison de la protéine 67 kD à l'élastine.

Au contraire, les oligosaccharides ne contenant pas de galactose sont incapables de permettre l'élution de la protéine 67 kD liée sur la colonne d'affinité élastine.

Les auteurs en ont donc conclu que le récepteur de l'élastine était une protéine ayant des propriétés de liaison aux galactosides.

Il est également connu que les élastines, à l'état soluble ou insoluble, sont susceptibles d'être hydrolysées par des endoprotéases et notamment les élastases.

Différentes classes de protéases et d'endopeptidases ont été répertoriées et une revue de ces différentes endopeptidases et de leurs propriétés a été publiée par Barrett et al., 1977 (Barrett A.J. Eds., North Holland, Amsterdam).

Les élastases sont des protéases et endopeptidases dont les propriétés commencent à être bien étudiées. Les articles suivants dont le contenu peut être incorporé dans le présent texte par référence décrivent ces propriétés, il s'agit de :

- Bieth J. (1978) "Elastase : structure, function and pathological role". Front. of Matrix Biol. Cis.: 1-82 et
- Homsy R. et al. (1988) "Characterization of human skin fibroblasts elastase activity". Journ. Invest. Dermatol. 91: 472-477.

La désignation d'une enzyme sous l'appellation d'élastase n'implique pas que cette protéase soit spécifique de l'élastine. En fait, les élastases hydrolysent une grande variété de substrats protéiques et l'élastase leucocytaire par exemple est capable de cliver pratiquement toutes les macromolécules du tissu conjonctif (Robert L. 1988, Path. Biol.; 36:1101-1107).

Ces protéases de type élastase sont capables de dégrader les fibres élastiques des tissus, ainsi d'ailleurs que d'autres constituants comme les fibres de collagène ou les glycoprotéines, par exemple la fibronectine. Ce type de phénomène a été décrit dans les phénomènes de vieillissement cellulaire ; de nombreux travaux ont montré que la synthèse et l'accumulation de ce type d'enzymes augmente au cours du vieillissement, on peut citer notamment :

- Labat-Robert J. et al., Ann. New York Acad. Sci. (1992), 673:16-22,
- Robert J. Sang Thrombose Vaisseaux (1991), 3:267-330,
- Robert L. Pathol. Biol. (1988), 36:1101-1107.

Ces mêmes protéases et endopeptidases sont également capables de dégrader les constituants matriciels d'autres organes, par exemple l'élastine des poumons, induisant ainsi un emphysème pulmonaire, ou encore de dégrader les lames élastiques des artères, et l'élastine et le collagène des veines, favorisant ainsi le développement de maladies vasculaires : athérosclérose et artériosclérose des artères, et varicoses des veines. Toutes ces pathologies voient leur fréquence et leur gravité augmenter avec l'âge.

D'autres endopeptidases de même nature (métallo-endopeptidases à Zn) sont capables d'activer l'angiotensine I inactive, en angiotensine II qui peut déclencher une hypertension artérielle sévère.

Enfin, les enképhalinases, d'autres endopeptidases à Zn de même nature, interfèrent avec le fonctionnement des enképhalines et, selon l'importance de leurs excès, peuvent induire des perturbations dans le fonctionnement du cerveau.

Le dénominateur commun de toutes ces endopeptidases agissant sur des substrats différents et induisant des pathologies très diverses est qu'elles possèdent un site actif à Zn et sont toutes liées à la membrane cellulaire.

Dans la description qui va suivre, le cas de l'élastase des fibroblastes de la peau sera traité, mais le mécanisme physiopathologique et le principe du traitement qui seront présentés sont également valables pour les autres endopeptidases et les autres pathologies dont l'énumération non limitative a été donnée ci-dessus.

La présente invention est relative à des compositions pour le traitement ou la prévention des manifestations du vieillissement du tissu conjonctif, caractérisées en ce qu'elles contiennent au moins un oligosaccharide de 2 à 5 résidus osidiques ou un dérivé de celui-ci, contenant un galactose en position terminale non réductrice.

L'invention concerne une composition caractérisée en ce qu'elle est utilisée pour le traitement des manifestations du vieillissement cutané, quand les oligosaccharides ou leurs dérivés sont associés à un véhicule compatible avec une administration cosmétique, et de préférence topique.

L'invention est également relative aux compositions pour le traitement ou la prévention des manifestations de vieillissements accélérés au niveau de la trame fibreuse du tissu conjonctif accompagnant certaines pathologies, notamment des pathologies cardio-vasculaires et l'emphysème pulmonaire, ladite composition contenant les oligosaccharides ou dérivés de ceux-ci ayant un galactose en position terminale non réductrice.

Les oligosaccharides entrant dans la composition pour le traitement ou la prévention des manifestations du vieillissement cutané ou de vieillissements accélérés au niveau de la trame fibreuse du tissu conjonctif sont, de préférence, le lactose, le mélibiose, le stacchiose ou des dérivés de l'un de ces sucres ou un mélange de ceux-ci.

Les dérivés d'oligosaccharides qui peuvent rentrer dans une composition selon l'invention comportent tous un résidu du galactose en position terminale non réductrice ; ils peuvent rentrer dans les catégories citées ci-après, dans lesquelles l'oligoside répond à la formule générale suivante :

$$\text{galactose-}_n(\alpha \text{ ou } \beta) - (\text{Hex})_{n'},$$

dans laquelle

n représente la position 1, 2, 3, 4 ou 6,
Hex représente un pentose ou un hexose en liaison $\alpha$ ou $\beta$,
n' est un nombre compris entre 1 et 5;

a)- des glycosides répondant aux formules :

- (I) oligoside 1-O-R, dans lequel R est un résidu alkyle de 1 à 18 atomes de carbone, linéaire ou branché,
- (II) oligoside 1-O-R-O-1-oligoside dans laquelle R = $(CH_2)_n$, n étant compris entre 2 et 10,

b)- une osylamine acylée selon l'une des formules suivantes, dans lesquelles l'oligoside est de préférence le lactose, le mélibiose ou le stachiose :

- des osylamines acylées répondant à l'une des formules suivantes :
  - (III) oligoside 1-NH-CO-R, dans laquelle R est un résidu alkyle de 2 à 18 atomes de carbone, contenant 0, 1 ou 2 double liaisons,
  - (IV) oligoside 1-NH-CO-R-CO-NH-1-oligoside, dans laquelle R = $(CH_2)_n$, n étant compris entre 2 et 8,

c)- une alkylamine acylée par un acide aldonique obtenu par oxydation d'un oligoside,

- (V) oligoside-CO-NH-R, dans laquelle R a la même signification que dans la formule (III),
- (VI) oligoside CO-NH-R-NH-CO-oligoside, où R a la même signification que dans la formule (III),

d)- soit un produit de réduction des bases de Schiff formé par des oligosides avec des mono- ou diamines aliphatiques, et répondant à l'une des formules suivantes :

- (VII) Gal-$(Hex)_n$-X-HN-R,
- (VIII) Gal-$(Hex)_n$-X-HN-R-NH-X-$(Hex)_n$-Gal,

dans lesquelles :

Hex est un hexose ou un pentose,

n = 0, 1 ou 2,
X = 1-$NH_2$-hexitol, et
R a la même signification que dans (III).

L'ensemble de ces oligosaccharides ou de leurs dérivés décrits plus haut ont en commun la caractéristique de diminuer d'au moins 20% la synthèse et/ou la sécrétion de l'élastase dans des fibroblastes en culture.

Cet effet se manifeste par la diminution de l'activité enzymatique dosable dans le milieu de culture de ces cellules, ainsi que dans les cellules elles-mêmes.

La méthode utilisée consiste à cultiver les fibroblastes de peau humaine dans des conditions standards et à déterminer par une méthode colorimétrique, à l'aide d'un substrat synthétique (le N-succinyl-trialanyl-paranitroanilide), l'activité élastasique de la culture.

En utilisant ce substrat synthétique, il a pu également être montré que la biosynthèse des endopeptidases de type élastase augmente au cours des passages successifs des fibroblastes constituant ainsi un système de vieillissement in vitro selon le modèle de Hayflick (1980, Cell Adding In. Annual Review of Gerontology and Geriatrics ; Springer, New York 1:26-67) ; Labat-Robert J. et al. (1988, Expert Gerontol. 23:5-18).

L'activité enzymatique est essentiellement associée avec la membrane cellulaire et peut être récupérée après trypsinisation des fibroblastes dans une fraction soluble.

Deux à 10% de l'activité totale peuvent être récupérés dans les milieux de culture et le reste est associé avec les résidus cellulaires et peuvent être solubilisés à l'aide d'un détergent.

La présente invention est relative à l'utilisation des oligosaccharides ou de leurs dérivés décrits plus haut pour contrôler la biosynthèse et la sécrétion des protéases de type élastase à partir de fibroblastes par le récepteur de l'élastine présent sur les fibroblastes humains, notamment les fibroblastes de la peau, ce contrôle ayant pour effet de prévenir ou traiter les manifestations du vieillissement cutané, ou celles du vieillissement accéléré lié aux pathologies dues notamment à une présence excessive d'endopeptidases.

En effet, comme il a été dit plus haut, la sous-unité de 67 kD du récepteur de l'élastine possède une structure réagissant spécifiquement avec le lactose ou des structures oligo- ou polysaccharidiques contenant du galactose en position terminale (Labat-Robert J. et al., cité ci-dessus et Mecham R.P. et al. 1989, Biochem. 28:3716-3722).

Les dérivés d'oligosaccharides entrant dans la composition de l'invention peuvent être synthétisés soit chimiquement, comme il sera expliqué plus loin dans les exemples, soit extraits à partir de milieux biologiques, notamment le lait, le sang ou le placenta, ou encore de produits naturels comme le miel qui contient des galactosides intéressants pour la préparation des compositions de l'invention (R. Siddiqui, in Advance in Carbohydrate Chemistry and Biochemistry, Ed: D. Horton vol. 49, pp. 285-309).

Les polysaccharides ou leurs dérivés peuvent ainsi être extraits par des méthodes classiques (Frontiers of Matrix Biology (1985), vol. 10, S. Kargar Edition), puis subir soit une dégradation enzymatique, soit une hydrolyse chimique et enfin le composé d'intérêt est séparé soit par précipitation saline, ou chromatographie d'échange d'ions ou chromatographie sur gel de Sepharose, ou par tout autre procédé adéquat.

La présente invention est également relative au procédé de préparation d'une composition destinée à la prévention ou au traitement des effets du vieillissement cutané caractérisé en ce qu'il comprend l'incorporation dans un véhicule acceptable pour une administration locale d'un oligosaccharide de 2 à 5 résidus osidiques comprenant un galactose en position terminale non réductrice ou d'un dérivé d'un tel oligosaccharide, lequel représente 0,5 à 6% en poids sur volume de ladite composition.

Cette composition peut être avantageusement utilisée en dermatologie et/ou cosmétologie pour le traitement ou la prévention des effets du vieillissement cutané.

De la même manière, l'invention est relative à un procédé de préparation d'une composition destinée à la prévention ou au traitement des manifestations de vieillissement accéléré au niveau de la trame fibreuse du tissu conjonctif accompagnant certaines pathologies liées à une synthèse anormale d'élastase, notamment l'emphysème pulmonaire, l'athérosclérose et artériosclérose des artères et varicoses des veines, ou enfin dans le traitement ou la prévention de l'hypertension artérielle lorsque des endopeptidases de type élastases sont responsables de l'activation de l'angiotensine I en angiotensine II ; cette utilisation est caractérisée par l'incorporation dans une composition, en association avec un véhicule pharmaceutique acceptable, d'un oligosaccharide ou un dérivé d'oligosaccharide comportant un galactoside en position terminale non réductrice.

Lorsque le dérivé d'oligosaccharides comprend un résidu hydrophobe, un véhicule avantageux peut être constitué de liposomes, permettant de présenter la fraction osidique du dérivé à leur surface.

Dans les exemples détaillés qui suivent, il est montré l'effet du lactose, du mélibiose du stachiose et de leurs dérivés sur la synthèse et la sécrétion d'élastase à partir de fibroblastes humains ayant subi de 15 à 25 passages : les cellules ainsi traitées sont des cellules qui entrent dans une phase de sénescence selon le modèle de Hayflick (référence supra) et constituent donc le modèle de vieillissement in vitro.

La diminution observée de la synthèse et de la sécrétion d'élastase dans un tel système par l'administration

des dérivés d'oligosaccharides de l'invention, est un indice de l'effet de ces composés anti-vieillissement puisque, comme nous l'avons vu plus haut, l'augmentation de la synthèse et de la sécrétion d'élastase sont des phénomènes liés au vieillissement de ces fibroblastes.

Ces exemples ne sont pas limitatifs d'un effet sur les fibroblastes de la peau, mais peuvent être généralisés à tout type de cellules fibroblastiques et autres cellules endothéliales, cellules musculaires lisses, leucocytes possédant dans leur membrane un récepteur de l'élastine, lequel récepteur comprend une sous-unité de 67 kD ayant une affinité pour les galactosides.

- Matériel et méthodes :

Les fibroblastes de la peau humaine ont été obtenus de biopsies de peau prélevée au cours d'une chirurgie plastique mammaire d'une femme âgée entre 16 et 24 ans.

Les cellules sont cultivées dans un milieu Eagle modifié par Dulbecco (DMEM) avec 10% de sérum de veau foetal, 200 μg/ml de pénicilline et 200 unités/ml de streptomycine dans une atmosphère 90/10% d'oxygène/azote dans des bouteilles en plastique NUNCLON-DELTA de 35x10 mm.

Les expériences sont réalisées entre le 18ème et le 21ème passage, dans la mesure où c'est à ce niveau là que l'activité élastasique augmente et refléte l'état de sénescence des cellules.

Vingt-quatre heures avant les expériences, le milieu est retiré et 1 ml de milieu DMEM frais a été ajouté avec du lactose à la concentration finale de 10 mM.

Après des intervalles de temps déterminés, le milieu a été enlevé, la couche de cellules rincée deux fois avec 1 ml de PBS (Phosphate Buffered Saline) qui a été ajouté au milieu.

La couche de cellules a ensuite été traitée avec une solution fraîche de trypsine (Worthingthon) à 20 μg dans 1 ml de PBS pendant 5 min. à 37°C, suivie par une centrifugation à 100 g pendant 5 min. à 4°C.

Après décantation et rinçage avec 1 ml de PBS, les résidus cellulaires sont suspendus dans 1 ml de tampon Tris-HCl 0,1 M pH 7.4, contenant 0,1% de Triton x-100 et homogénéisés dans un homogénéiseur Potter.

Le surnageant de cette suspension a été utilisé pour la détermination de l'activité enzymatique liée aux cellules.

La détermination de l'activité enzymatique de type élastase a été faite par addition du substrat (N-succinyl-trialanyl-paranitroanilide ou N-suc-ala$_3$p-Na) à une concentration de 125 mM dans le diméthylformamide à un aliquot d'extraits cellulaires selon la technique décrite dans Bieth. J. 1978 (voir supra).

Pour le milieu de culture, 56 μl ont été ajoutés à 890 μl de tampon Tris-HCl 0,1 M pH 7.8 et 10 μl de la solution de substrat.

Cinquante μl du trypsinat ou de résidus cellulaires ont été ajoutés à 940 μl de tampon Tris et à 10 μl de substrat.

Les incubations sont réalisées à 37°C pendant 3 h pour le milieu de culture, 1 h pour le trypsinat et pour le résidu cellulaire.

La densité optique de la couleur jaune qui se développe par action de l'enzyme sur le substrat est lue à 410 nanomètres dans un spectrophotomètre.

Toutes les expériences sont réalisées sur 4 à 6 cultures en parallèle, et des valeurs moyennes sont comparées statistiquement avec le t-test de Student.

L'activité enzymatique (AE) est exprimée en nM de substrat hydrolysé par heure et par $10^6$ cellules, calculée de la façon suivante :

$$AE = \frac{DO \text{ x v total}}{8{,}8 \text{ x v échantillon x } N \text{ x } T} \text{ x } 10^6$$

où :

- DO est la densité optique,
- N est le nombre de cellules dans le volume réactionnel,
- T est le temps d'incubation, et
- 8,8 est le coefficient d'extinction molaire correspondant à la DO de la solution molaire de substrat dans une cuve de 1 cm d'épaisseur.

Dans les différents exemples qui suivent, l'effet du galactose et de ses dérivés sur la modification de l'activité élastasique des fibroblastes a été étudié en mesurant la modification de cette activité par un peptide de l'élastine : la kappa-élastine (KE) ; ce peptide a été obtenu par une hydrolyse d'élastine du ligament nuqual du boeuf, par hydrolyse alcaline dans l'éthanol aqueux selon la technique décrite dans Robert L. et Poullain N. (Etudes sur la structure de l'élastine et le mode d'action de l'élastase. I. Nouvelle méthode de préparation de dérivés solubles de l'élastine. Bull.

Soc. Chim. Biol. 45:1317-1326 (1963)).

Ce peptide a en effet la propriété d'être un agoniste du récepteur de l'élastine, c'est-à-dire que sa présence conduit à une augmentation de l'activité élastase des fibroblastes en culture.

Le tableau 1 ci-dessous montre la cinétique d'actions de la kappa-élastine à 1 mg/lamelle sur l'activité élastasique des fibroblastes humains au vingtième passage en fonction du temps d'incubation.

Tableau 1

| TEMPS D'INCUBATION AVEC LA KE | | | | | |
|---|---|---|---|---|---|
| | 0 h | 2 h | 6 h | 24 h | 48 h |
| AE DMEM | 0,04 | 0,04 | 0,03 | 0,21 | 0,99 |
| AE TRYPS | 0,267 | 0,2 | 0,207 | 0,190 | 0,123 |

On note une forte augmentation de l'activité élastasique dans le milieu de culture (sécrétion) après un temps de latence de 24 h. En même temps, l'activité membranaire dans le trypsinat baisse. Il apparaît donc que l'augmentation de l'activité dans le milieu pourrait être liée au relargage de l'enzyme membranaire dans le milieu.

- EXEMPLE 1 : Etude de l'action du lactose :

La concentration utilisée de lactose est de 10 mM ou de 3,6 mg/ml. Le tableau 2 ci-dessous montre l'effet du lactose 10 mM sur l'activité élastase des fibroblastes humains au 21ème passage et sur sa distribution entre la forme libre, membranaire (trypsinat) et liée à la cellule (après effet du Triton).

L'activité est indiquée en densité optique du substrat hydrolysé, multipliée par $10^3$/h pour $10^6$ cellules.

Une moyenne de 4 mesures parallèles sur des cultures différentes a été calculée.

La distribution de l'activité entre les trois compartiments est indiquée comme un pourcentage du total, de même que l'inhibition par le lactose est indiquée en pourcentage de l'activité équivalente en l'absence de lactose.

Tableau 2

| | Contrôle | | Lactose | | |
|---|---|---|---|---|---|
| | Activité | % du total | Activité | % du total | % |
| Activité totale | 588.1 | 100 | 262.2 | 100 | 55 |
| Milieu de culture | 56.1±7.6 | 10 | 18.8±1.1 | 7 | 66 |
| Trypsinat | 359.5±41.7 | 61 | 91.9±31.7* | 35 | 74 |
| Cellulaire | 172.5±16.8 | 29 | 151.5±28.8 | 58 | 12 |

* $p < 0.05$ en comparant le contrôle avec le lactose.

Ce tableau montre une réduction de 55% de l'activité totale, ainsi qu'un changement de la distribution de l'activité enzymatique dans les trois fractions.

L'enzyme libre mesurée dans le milieu de culture diminue de 66% comparé à la culture contrôle et l'enzyme associée à la membrane relargable par la trypsine baisse de 74% tandis que l'activité résiduelle (cellulaire) ne montre pas de différence significative avec le contrôle.

Cette inhibition sélective des activités liées à la membrane et relarguées dans le milieu modifie la distribution de l'activité totale.

Dans les cultures contrôles, la majeure partie de l'activité totale est associée à la membrane cellulaire (61%) et relargable par la trypsine ; dans les cultures traitées au lactose, seulement 35% de l'activité totale sont relargués par la trypsine ; 58% sont associés aux résidus cellulaires.

Il apparaît ainsi que le lactose a pour effet de diminuer la synthèse de l'enzyme et le relargage.

- EXEMPLE 2 : Etude de l'action du mélibiose sur la synthèse d'élastase :

Le mélibiose est ajouté au milieu de culture à la concentration de 10 mM, soit 3,4 mg/ml et ce, dans les mêmes conditions de culture que le lactose.

Le mélibiose provoque une diminution de l'activité enzymatique dans le milieu de 60% par rapport au témoin et une diminution dans le trypsinat de 80%, ainsi qu'une faible diminution dans le culot cellulaire.

L'activité enzymatique totale est diminuée de 50%.

- EXEMPLE 3 : Action du stachiose :

Le stachiose est un tétrasaccharide qui a été préparé selon la technique de M.L. Wolfrom et A. Thompson décrite dans Methods of Carbohydrate Chemistry I p. 368-369, Academic Press, 1962.
Le stachiose a été ajouté au milieu de culture à la concentration de 10 mM, soit 6,6 mg/ml.
Il a pour effet de diminuer l'activité enzymatique dans le trypsinat de 30% par rapport au témoin, alors qu'il augmente légèrement dans le milieu.
En présence de kappa-élastine à la concentration de 1 mg/ml, il diminue l'activité enzymatique significativement dans le milieu (52%) par rapport à l'effet de la kappa-élastine seule.
Dans le trypsinat, on observe une augmentation de 32% toujours par rapport à l'effet de la kappa-élastine seule.
L'activité enzymatique dans les culots ne varie pas d'une façon significative.

- EXEMPLE 4 : Action de l'oleoyl-lactosylamine :

L'oleoyl-lactosylamine est un composé répondant à la formule X et sa synthèse est nouvelle.

a) **Synthèse de l'oleoyl-lactosylamine :**

Du 1-amino lactose réagit avec du l'oleoyl-chlorure pour donner le lactobionyl-oléylamine de formule suivante :

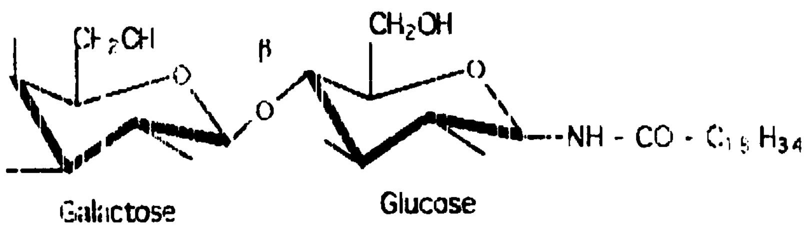

On dissout 3,3 g de lactosylamine dans 30 ml de méthanol à 80° et on y ajoute 1,2 g de diéthylamine. On ajoute à cette solution 4 g de chlorure d'oleoyl, sous agitation, à une température ne dépassant pas 30°C.
Le pH du mélange est porté à 3 par l'addition de HCl. L'acide oléique est éliminé par extraction par heptane. On fait passer la phase méthanolique-aqueuse successivement par les colonnes d'échangeur d'ions acides et basiques et on chasse les solvants sous vide.
Le produit obtenu renferme encore environ 20% de lactose. L'oléoyl-lactosylamine peut être purifié sur une colonne d'octyl-Sepharose.
Rf = sur silice (MeOH couche mince), 0,8 (chloroforme-méthanol 1:1, v/v).

b) **Effet sur les cultures sénescentes de fibroblastes :**

L'oleoyl-lactosylamine est ajouté à la concentration de 0,5 mM.
A cette concentration, il provoque une faible augmentation de l'activité dans le trypsinat et une baisse dans les culots cellulaires d'environ 20%.

- EXEMPLE 5 : Action du dimélibionityl-diaminohexane :

a) **Préparation du dimélibionityl-diaminohexan:**

Ce produit a été synthétisé par une technique nouvelle décrite ci-après.
On dissout 374 mg mélibiose (1,1 mM) et 116 mg (0,5 mM) de diaminohexane dans 5 ml de tampon phosphate 0,2 M pH 8, et on y ajoute 270 mg de cyanoborohydrure de sodium (4,4 mM). On abandonne le mélange 11 jours à la température ambiante, et on chauffe à 37°C pendant 2 jours. On fait passer la solution sur échangeur d'ions acides (H+) et on lave la colonne par l'eau.

La substance est éluée ensuite par une solution de 0,5 M d'ammoniaque, et on lyophilise la solution. Le rendement est de 200 mg de di-mélibionityle-diaminohexane.
Rf sur silice = (Butanol-acide acétique-eau, h = 1:1) 0,2.
La figure 1 représente le schéma réactionnel conduisant aux dérivés dimélibionityl-diaminohexanes.

b) **Action du diméliobionityl-diaminohexane sur l'activité enzymatique des cultures de fibroblastes humains :**

On observe une augmentation de l'activité enzymatique dans le milieu, ainsi qu'une diminution de 40% dans le trypsinat par rapport au témoin.
En présence de kappa-élastine, l'activité diminue de 71% dans le milieu, sans augmentation dans le trypsinat.
Par contre, l'activité récupérée dans les culots cellulaires augmente légèrement par rapport à l'action de la kappa-élastine seule, mais ne déplace pas la valeur du témoin.

c) **Comparaison des différents effets :**

Le tableau 3 suivant résume l'ensemble des résultats obtenus avec les différents dérivés galactosides sur l'activité élastasique des fibroblastes humains :

TABLEAU IV :

| ACTION SUR L'ACTIVITE ELASTASIQUE | | | | |
|---|---|---|---|---|
| SUBSTANCE | TOTAL | DMEM | TRYPSINAT | CULOTS C. |
| LACTOSE | ↓27% | ↓30% | ↓70% | ↓30% |
| LACTOSE+KE | ↑13,5% | ↑20% | ↑50% | ↓30% |
| MELIBIOSE | ↓58% | ↓60% | ↓80% | ↓30% |
| MELIBIOSE+KE | ↑15% | ↓27% | ↑31% | ↑9% |
| STACHIOSE | ↓17% | ↑14% | ↓35% | - |
| STACHIOSE+KE | ↓17% | ↓56% | ↑32% | - |
| CARRAGEENAN | ↑28% | - | ↑66% | ↑30% |
| CARRAGEENAN+KE | - | ↓28% | ↑29% | - |
| L.O.AMINE | ↓15% | ↑44% | ↑61% | ↓19% |
| L.O.AMINE+KE | ↓27% | - | ↑41% | ↓32% |
| M.L.AMINE | ↑13% | ↓10% | ↓15% | ↑50% |
| M.L.AMINE+KE | ↑40% | ↑11% | ↑17% | ↑50% |
| AMB | ↓23% | ↑67% | - | ↓35% |
| AMB+KE | ↓7% | ↓60% | ↓20% | ↓23% |
| DMDAH | - | ↑67% | ↓53% | - |
| DMDAH+KE | ↑23% | ↑70% | ↓18% | ↑34% |
| MDH | ↑58% | ↓60% | ↑37% | ↑60% |
| MDH+KE | ↑54% | ↓80% | ↑14% | ↑56% |
| **LOAMINE-** LACTOBIONYL-OLEYLAMINE<br>**MLAMINE-** MYRISTOYL-LACTOSYLAMINE<br>**AMB-** ACIDE MELIBIONIQUE<br>**DMDAH-** DIMELIBIONITOYL-DIAMMINOHEXANE<br>**MDH-** DIMELIBIITYL-DIAMINOHEXANE | | | | |

Les dérivés soulignés sont ceux qui sont indiqués dans les exemples précédents.

- EXEMPLE 6 : Effet chez le rat nu du lactose et du méliobiose :

Deux groupes de rats sont traités et comparés, l'un avec le lactose ou le mélibiose, l'autre par un placebo.
Puis, les rats sont irradiés avec une dose de 3 MED (pour dose minimale érythémateuse).
Le dosage d'activité élastasique est mesurée au niveau de biopsies prélevées sur les rats irradiés, par la

méthode décrite dans Matériels et méthode.

**Revendications**

1. Utilisation d'au moins un oligosaccharide comprenant de 2 à 5 résidus osidiques ou d'un dérivé de celui-ci contenant un résidu hydrophobe, ledit oligosaccharide comportant un résidu galactose en position terminale non réductrice, pour la préparation d'un médicament utile pour le traitement ou la prévention du vieillissement du tissu conjonctif.

2. Utilisation selon la revendication 1 d'un oligosaccharide comprenant de 2 à 5 résidus osidiques ou d'un dérivé de celui-ci contenant un résidu hydrophobe, ledit oligosaccharidc comportant un résidu galactose en position terminale non réductrice pour la préparation d'un médicament capable d'inhiber le récepteur membranaire de l'élastine Impliqué dans la dégradation de la trame fibreuse du tissu conjonctif.

3. Utilisation d'au moins un oligosaccharide comprenant de 2 à 5 résidus osidiques, ou d'un dérivé de celui-ci contenant un résidu hydrophobe, pourvu qu'un résidu galactose soit présent en position terminale non réductrice de l'oligosaccharide pour le traitement cosmétique des manifestations du vieillissement du tissu conjonctif.

4. Utilisation d'un oligosaccharide selon l'une des revendications 1 et 2, caractérisée en ce que l'oligosaccharide ou ses dérivés sont associés à un véhicule compatible avec une administration topique, pour la préparation d'un médicament utile pour la prévention ou le traitement des manifestations du vieillissement cutané.

5. Utilisation d'un oligosaccharide selon la revendication 3, caractérisée en ce que l'oligosaccharide ou ses dérivés sont associés à un véhicule compatible avec une administration topique, pour le traitement cosmétique des manifestations du vieillissement cutané.

6. Utilisation selon l'une des revendications 1 à 5, caractérisée en ce que l'oligosaccharide présente la formule suivante :

$$\text{galactose-}_{n}(\alpha \text{ ou } \beta) - (X)_{n'}$$

dans laquelle

n représente la position 1, 2, 3, 4 ou 6,
X représente un pentose ou un hexose en liaison $\alpha$ ou $\beta$,
n' est un nombre compris entre 1 et 5.

7. Utilisation d'un oligosaccharide selon la revendication 6, caractérisée en ce que l'oligosaccharide est le mélibiose.

8. Composition pharmaceutique, caractérisée en ce qu'elle contient à titre de principe actif un oligosaccharide ou un dérivé d'oligosaccharide sur lequel est greffé un radical hydrophobe, en mélange avec un excipient pharmaceutiquement acceptable, avec l'oligosaccharide répondant à la formule générale suivante :

$$\text{galactose-}_{n}(\alpha \text{ ou } \beta) - (X)_{n'} \qquad \text{(A)}$$

dans laquelle

n représente la position 1, 2, 3, 4 ou 6,
X représente un pentose ou un hexose en liaison $\alpha$ ou $\beta$,
n' est un nombre compris entre 1 et 5,

à la condition que si l'oligosaccharide n'est pas substitué, il est différent du lactose,
et en ce que le dérivé d'oligosaccharide est choisi dans l'une des catégories suivantes :

a)- un glycoside selon l'une des formules suivantes, dans lesquelles l'oligosaccharide est un oligoside de préférence le lactose, le mélibiose ou le stachiose :

- (I) oligoside 1-0-R, dans lequel R est un résidu alkyle de 1 à 18 atomes de carbone, linéaire ou branché, à la condition que
  - si dans la formule générale (A), X représente gal et n vaut 1, R est différent de éthyl ou méthyl,
  - si l'oligosaccharide est le lactose, R est différent d'un résidu alkyle de 1 à 6 atomes de carbone,
- (II) oligoside 1-0-R-O-1-oligoside dans laquelle
  R = $(CH_2)_n$, n étant compris entre 2 et 10,

b)- une osylamine acylée selon l'une des formules suivantes, dans lesquelles l'oligoside est de préférence le lactose, le mélibiose ou le stachiose :

- (III) oligoside 1-NH-CO-R, dans laquelle
  R est un résidu alkyle de 2 à 18 atomes de carbone, contenant 0, 1 ou 2 double liaisons,
- (IV) oligoside 1-NH-CO-R-CO-NH-1-oligoside, dans laquelle
  R = $(CH_2)_n$, n étant compris entre 2 et 8,

c)- une alkylamine acylée par un acide aldonique obtenu par oxydation d'un oligoside,

- (V) oligoside-CO-NH-R, dans laquelle
  R a la même signification que dans la formule (III),
- (VI) oligoside CO-NH-R-NH-CO-oligoside, où R a la même signification que dans la formule (III),

d)- soit un produit de réduction des bases de Schiff formé par des oligosides avec des mono- ou diamines aliphatiques, et répondant à l'une des formules suivantes :

- (VII) Gal-$(Hex)_n$-X-HN-R,
- (VIII) Gal-$(Hex)_n$-X-HN-R-NH-X-$(Hex)_n$-Gal,
  dans lesquelles :

  Hex est un résidu d'un hexose (ou pentose),
  n = 0,1 ou 2,
  X = résidu d'un hexitol (ou pentitol), et
  R a la même signification que dans (III).

**9.** Composition selon la revendication 8, caractérisée en ce qu'elle contient à titre de principe actif au moins un oligosaccharide choisi parmi le mélibiose, le stachiose ou leurs mélanges.

**10.** Composition cosmétique, caractérisée en ce qu'elle contient, à titre d'agent actif sur le vieillissement du tissu conjonctif, un composé choisi dans le groupe constitué du mélibiose, et des dérivés d'oligosaccharides comportant de 2 à 5 résidus osidiques et un galactose en position terminale non réductrice, substitués par un radical hydrophobe, les dérivés étant choisis parmi :

a)- un glycoside selon l'une des formules suivantes, dans lesquelles l'oligosaccharide est un oligoside de préférence le lactose, le mélibiose ou le stachiose :

- (I) oligoside 1-0-R, dans lequel R est un résidu alkyle de 1 à 18 atomes de carbone, linéaire ou branché,
- (II) oligoside 1-0-R-O-1-oligoside dans laquelle
  R = $(CH_2)_n$, n étant compris entre 2 et 10,

b)- une osylamine acylée selon l'une des formules suivantes, dans lesquelles l'oligoside est de préférence le lactose, le mélibiose ou le stachiose :

- (III) oligoside 1-NH-CO-R, dans laquelle
  R est un résidu alkyle de 2 à 18 atomes de carbone, contenant 0, 1 ou 2 double liaisons,

. (IV) oligoside 1-NH-CO-R-CO-NH-1-oligoside, dans laquelle
R = $(CH_2)_n$, n étant compris entre 2 et 8,

c)- une alkylamine acylée par un acide aldonique obtenu par oxydation d'un oligoside,

. (V) oligoside-CO-NH-R, dans laquelle
R a la même signification que dans la formule (III),
. (VI) oligoside CO-NH-R-NH-CO-oligoside, où R a la même signification que dans la formule (III),

d)- soit un produit de réduction des bases de Schiff formé par des oligosides avec des mono- ou diamines aliphatiques, et répondant à l'une des formules suivantes :

. (VII) Gal-$(Hex)_n$-X-HN-R,
. (VIII) Gal-$(Hex)_n$-X-HN-R-NH-X-$(Hex)_n$-Gal,
dans lesquelles :

Hex est un résidu d'un hexose (ou pentose),
n = 0, 1 ou 2,
X = résidu d'un hexitol (ou pentitol), et
R a la même signification que dans (III).

**11.** Composition dermo-cosmétique, caractérisée en ce qu'elle contient du mélibiose.

**12.** Procédé de préparation d'une composition selon l'une des revendications 8 à 10, caractérisé en ce que les dérivés d'oligosaccharides sont obtenus par synthèse chimique.

**13.** Procédé de préparation d'une composition selon l'une des revendications 8 à 11, caractérisé en ce qu'il comprend les étapes suivantes:

a) extraction des sucres à partir d'un milieu biologique, notamment le lait, le sang ou le placenta,
b) dégradation enzymatique des sucres extraits ou hydrolyse chimique,
c) séparation de l'oligosaccharide recherché.

**14.** Procédé de préparation d'une composition selon l'une des revendications 8 à 11 destinée à la prévention ou au traitement des effets du vieillissement, caractérisé en ce qu'il comprend l'incorporation dans un véhicule acceptable pour une administration locale d'un oligosaccharide contenant de 2 à 5 résidus osidiques comprenant un galactose en position terminale non réductrice ou d'un dérivé d'un tel oligosaccharide, lequel représente 0,5% à 6%, de préférence 1 à 4%, en poids sur volume de ladite composition.

## Claims

**1.** Use of at least one oligosaccharide comprising 2 to 5 glycosidic residues, or of a derivative of such an oligosaccharide containing a hydrophobic residue, the said oligosaccharide containing a galactose residue in a non-reducing terminal position, for the preparation of a medicinal product which is useful for treatment or prevention of the aging of connective tissue.

**2.** Use according to Claim 1 of an oligosaccharide comprising 2 to 5 glycosidic residues, or of a derivative of such an oligosaccharide containing a hydrophobic residue, the said oligosaccharide containing a galactose residue in a non-reducing terminal position, for the preparation of a medicinal product capable of inhibiting the membrane receptor for elastin which is involved in the degradation of the fibrous stroma of the connective tissue.

**3.** Use of at least one oligosaccharide comprising 2 to 5 glycosidic residues, or of a derivative of such an oligosaccharide containing a hydrophobic residue, provided that a galactose residue is present in a non-reducing terminal position of the oligosaccharide, for the cosmetic treatment of the manifestations of the aging of connective tissue.

**4.** Use of an oligosaccharide according to one of Claims 1 and 2, characterized in that the oligosaccharide or its derivatives are combined with a vehicle that is compatible with a topical administration, for the preparation of a

medicinal product which is useful for the prevention or treatment of the manifestations of skin aging.

**5.** Use of an oligosaccharide according to claim 3, characterized in that the oligosaccharide or its derivatives are combined with a vehicle that is compatible with a topical administration, for the cosmetic treatment of the manifestations of skin aging.

**6.** Use according to one of Claims 1 to 5, characterized in that the oligosaccharide possesses the following formula:

$$\text{galactose-}_n(\alpha \text{ or } \beta) - (X)_{n'}$$

in which

n represents position 1, 2, 3, 4 or 6,
X represents a pentose or hexose linked in the α or β configuration,
n' is a number between 1 and 5.

**7.** Use of an oligosaccharide according to Claim 6, characterized in that the oligosaccharide is melibiose.

**8.** Pharmaceutical composition, characterized in that it contains as active principle an oligosaccharide or an oligosaccharide derivative onto which a hydrophobic radical is grafted, mixed with a pharmaceutically acceptable excipient, with the oligosaccharide corresponding to the general formula:

$$\text{galactose-}_n(\alpha \text{ or } \beta) - (X)_{n'} \qquad (A)$$

in which

n represents position 1, 2, 3, 4 or 6,
X represents a pentose or hexose linked in the α or β configuration,
n' is a number between 1 and 5,

provided that, if the oligosaccharide is not substituted, it is other than lactose,
and in that the oligosaccharide derivative is chosen from one of the following categories:

a)- a glycoside according to one of the following formulae, in which the oligosaccharide is an oligoglycoside, preferably lactose, melibiose or stachyose:

- (I) oligoglycoside 1-0-R, in which R is a linear or branched alkyl residue with 1 to 18 carbon atoms, provided that
  - if in the general formula (A), X represents gal and n equals 1, R is other than ethyl or methyl,
  - if the oligosaccharide is lactose, R is other than an alkyl residue with 1 to 6 carbon atoms,
- (II) oligoglycoside 1-0-R-O-1-oligoglycoside in which
  $R = (CH_2)_n$, n being between 2 and 10,

b)- an acylated glycosylamine according to one of the following formulae, in which the oligoglycoside is preferably lactose, melibiose or stachyose:

- (III) oligoglycoside 1-NH-CO-R, in which
  R is an alkyl residue with 2 to 18 carbon atoms, containing 0, 1 or 2 double bonds,
- (IV) oligoglycoside 1-NH-CO-R-CO-NH-1-oligoglycoside, in which
  $R = (CH_2)_n$, n being between 2 and 8,

c)- an alkylamine acylated with an aldonic acid obtained by oxidation of an oligoglycoside,

- (V) oligoglycoside-CO-NH-R, in which R has the same meaning as in the formula (III),
- (VI) oligoglycoside CO-NH-R-NH-CO-oligoglycoside, where R has the same meaning as in the formula (III),

d) - or a product of the reduction of the Schiff's bases which is [sic] formed by oligoglycosides with aliphatic mono- or diamines, and corresponding to one of the following formulae:

- (VII) Gal-$(Hex)_n$-X-HN-R,
- (VIII) Gal-$(Hex)_n$-X-HN-R-NH-X-$(Hex)_n$-Gal, in which:

  Hex is a residue of a hexose (or pentose),
  n = 0, 1 or 2,
  X = residue of a hexitol (or pentitol), and
  R has the same meaning as in (III).

**9.** Composition according to Claim 8, characterized in that it contains as active principle at least one oligosaccharide chosen from melibiose, stachyose and mixtures thereof.

**10.** Cosmetic composition, characterized in that it contains, as an agent which is active against the aging of connective tissue, a compound chosen from the group consisting of melibiose and oligosaccharide derivatives containing 2 to 5 glycosidic residues and a galactose in a non-reducing terminal position, such derivatives being substituted with a hydrophobic radical, the derivatives being chosen from:

a)- a glycoside according to one of the following formulae, in which the oligosaccharide is an oligoglycoside, preferably lactose, melibiose or stachyose:

- (I) oligoglycoside 1-0-R, in which R is a linear or branched alkyl residue with 1 to 18 carbon atoms,
- (II) oligoglycoside 1-0-R-O-1-oligoglycoside in which $R = (CH_2)_n$, n being between 2 and 10,

b)- an acylated glycosylamine according to one of the following formulae, in which the oligoglycoside is preferably lactose, melibiose or stachyose:

- (III) oligoglycoside 1-NH-CO-R, in which R is an alkyl residue with 2 to 18 carbon atoms, containing 0, 1 or 2 double bonds,
- (IV) oligoglycoside 1-NH-CO-R-CO-NH-1-oligoglycoside, in which $R = (CH_2)_n$, n being between 2 and 8,

c)- an alkylamine acylated with an aldonic acid obtained by oxidation of an oligoglycoside,

- (V) oligoglycoside-CO-NH-R, in which R has the same meaning as in the formula (III),
- (VI) oligoglycoside CO-NH-R-NH-CO-oligoglycoside, where R has the same meaning as in the formula (III),

d)- or a product of the reduction of the Schiff's bases which is formed by oligoglycosides with aliphatic mono- or diamines, and corresponding to one of the following formulae:

- (VII) Gal-$(Hex)_n$-X-HN-R,
- (VIII) Gal-$(Hex)_n$-X-HN-R-NH-X-$(Hex)_n$-Gal, in which:

  Hex is a residue of a hexose (or pentose),
  n = 0, 1 or 2,
  X = residue of a hexitol (or pentitol), and
  R has the same meaning as in (III).

**11.** Dermocosmetic composition, characterized in that it contains melibiose.

**12.** Process for preparing a composition according to one of Claims 8 to 10, characterized in that the oligosaccharide derivatives are obtained by chemical synthesis.

**13.** Process for preparing a composition according to one of Claims 8 to 11, characterized in that it comprises the following steps:

a) extraction of the sugars from a biological medium, especially milk, blood or placenta,
b) enzymatic degradation of the extracted sugars, or chemical hydrolysis,
c) separation of the desired oligosaccharide.

**14.** Process for preparing a composition according to one of Claims 8 to 11, intended for the prevention or treatment of the effects of aging, characterized in that it comprises the incorporation into an acceptable vehicle for local administration of an oligosaccharide containing 2 to 5 glycosidic residues comprising a galactose in a non-reducing terminal position, or of a derivative of such an oligosaccharide, which represents 0.5% to 6% and preferably 1 to 4% weight/volume of the said composition.

## Patentansprüche

**1.** Verwendung wenigstens eines Oligosaccharides, das 2 bis 5 Zuckerreste umfaßt, oder eines Derivates davon, das einen hydrophoben Rest enthält, wobei das Oligosaccharid einen Galactoserest an einem nicht-reduzierenden Ende umfaßt, für die Herstellung eines Medikaments, das nützlich ist für die Behandlung oder die Prävention der Alterung des Bindegewebes.

**2.** Verwendung eines Oligosaccharides nach Anspruch 1, das 2 bis 5 Zuckerreste umfaßt, oder eines Derivates davon, das einen hydrophoben Rest enthält, wobei das Oligosaccharid einen Galactoserest an einem nicht-reduzierenden Ende umfaßt, für die Herstellung eines Medikaments, das in der Lage ist, den Membranrezeptor des Elastins zu inhibieren, der am Abbau der fibrillären Matrix des Bindegewebes beteiligt ist.

**3.** Verwendung wenigstens eines Oligosaccharides, das 2 bis 5 Zuckerreste umfaßt, oder eines Derivates davon, das einen hydrophoben Rest enthält, vorausgesetzt, daß ein Galactoserest an einem nicht-reduzierenden Ende des Oligosaccharids vorhanden ist, für die kosmetische Behandlung von Manifestationen der Alterung des Bindegewebes.

**4.** Verwendung eines Oligosaccharids nach einem der Ansprüche 1 und 2, dadurch gekennzeichnet, daß das Oligosaccharid oder seine Derivate mit einem Vehikel verbunden sind, das für eine topische Verabreichung geeignet ist, für die Herstellung eines Medikaments, das für die Prävention oder Behandlung von Manifestationen der Hautalterung nützlich ist.

**5.** Verwendung eines Oligosaccharids nach Anspruch 3, dadurch gekennzeichnet, daß das Oligosaccharid oder seine Derivate mit einem Vehikel assoziiert sind, das für eine topische Verabreichung geeignet ist, für die kosmetische Behandlung der Manifestationen der Hautalterung.

**6.** Verwendung nach einem der Ansprüche 1 bis 5, dadurch gekennzeichnet, daß das Oligosaccharid die folgende Formel aufweist:

$$\text{Galactose-}_n(\alpha \text{ oder } \beta) - (X)_{n'}$$

worin

n die Positionen 1, 2, 3, 4 oder 6 darstellt,
X eine Pentose oder eine Hexose in α- oder β-Bindung darstellt,
n' eine Zahl zwischen 1 und 5 ist.

**7.** Verwendung eines Oligosaccharids nach Anspruch 6, dadurch gekennzeichnet, daß das Oligosaccharid die Melibiose ist.

**8.** Pharmazeutische Zusammensetzung, dadurch gekennzeichnet, daß sie als Wirkstoff ein Oligosaccharid oder ein Oligosaccharidderivat, auf dem ein hydrophobes Radikal aufgepfropft ist, zusammen mit einem pharmazeutisch akzeptablen Trägerstoff enthält, wobei das Oligosaccharid die folgende allgemeine Formel aufweist:

$$\text{Galactose-}_n(\alpha \text{ oder } \beta) \text{ - } (X)_{n'}$$

worin

n die Positionen 1, 2, 3, 4 oder 6 darstellt,
X eine Pentose oder eine Hexose in α- oder β-Bindung darstellt,
n' eine Zahl zwischen 1 und 5 ist,

unter der Bedingung, daß, wenn das Oligosaccharid nicht substituiert ist, es keine Lactose ist,
und sofern das Oligosaccharidderivat ausgewählt ist aus einer der folgenden Gruppen:

a) - ein Glycosid nach einer der folgenden Formeln, worin das Oligosaccharid ein Oligosid ist, bevorzugterweise die Lactose, die Melibiose oder die Stachiose:

- (I) Oligosid 1-O-R, worin R ein Alkylrest mit 1 bis 18 Kohlenstoffatomen, linear oder verzweigt, ist; unter der Voraussetzung, daß,
  - wenn in der allgemeinen Formel (A) X gal und n den Wert 1 aufweist, R verschieden ist von Ethyl oder Methyl,
  - wenn das Oligosaccharid die Lactose ist, R verschieden ist von einem Alkylrest mit 1 bis 6 Kohlenstoffatomen,
- (II) Oligosid 1-O-R-O-1-oligosid, worin
  $R = (CH_2)_n$ ist und n zwischen 2 und 10 enthalten ist,

b) - ein acyliertes Osylamin nach einer der folgenden Formeln ist, in denen das Oligosid bevorzugterweise die Lactose, die Melibiose oder die Stachiose ist:

- (III) Oligosid 1-NH-CO-R, worin
  R ein Alkylrest von 2 bis 18 Kohlenstoffatomen ist, der 0, 1 oder 2 Doppelbindungen enthält,
- (IV) Oligosid 1-NH-CO-R-CO-NH-1-Oligosid, worin
  $R = (CH_2)_n$ ist und n zwischen 2 und 8 enthalten ist,

c) - ein Alkylamin ist, das durch eine Aldonsäure acyliert ist, die durch Oxidation eines Oligosides erhalten ist,

- (V) Oligosid-CO-NH-R, worin
  R die gleiche Bedeutung wie in Formel (III) aufweist,
- (VI) Oligosid CO-NH-R-NH-CO-Oligosid, wo R die gleiche Bedeutung wie in Formel (III) aufweist,

d) - oder ein Reduktionsprodukt von Schiffschen Basen, das durch die Oligoside mit aliphatischen Mono- oder Diaminen gebildet ist, und eine der folgenden Formeln aufweist:

- (VII) Gal-$(Hex)_n$-X-HN-R,
- (VIII) Gal-$(Hex)_n$-X-HN-R-NH-X-$(Hex)_n$-Gal,
  worin:

  Hex ein Hexose- (oder Pentose-) Rest ist,
  n = 0, 1 oder 2,
  X = ein Hexitol- (oder Pentitol-) Rest ist, und
  R die gleiche Bedeutung aufweist wie in (III).

**9.** Zusammensetzung nach Anspruch 8, dadurch gekennzeichnet, daß sie als Wirkstoff wenigstens ein Oligosaccharid aufweist, das ausgewählt ist aus Melibiose, Stachiose oder ihren Mischungen.

**10.** Kosmetische Zusammensetzung, dadurch gekennzeichnet, daß sie als für die Alterung des Bindegewebes wirksames Agens eine Verbindung enthält, die ausgewählt ist aus der Gruppe, die Melibiose und Oligosaccharidderivate, die 2 bis 5 Zuckerreste und eine Galactose an einem nicht-reduzierenden Ende umfassen, substituiert durch ein hydrophobes Radikal, umfaßt, wobei die Derivate ausgewählt sind aus:

a) - einem Glycosid nach einer der folgenden Formeln, worin das Oligosaccharid ein Oligosid ist, bevorzugterweise die Lactose, die Melibiose oder die Stachiose:

. (I) Oligosid 1-O-R, worin R ein Alkylrest mit 1 bis 18 Kohlenstoffatomen, linear oder verzweigt, ist,
. (II) Oligosid 1-O-R-O-1-Oligosid, worin
R = $(CH_2)_n$ ist und n zwischen 2 und 10 enthalten ist,

b) - einem acetylierten Osylamin nach einer der folgenden Formeln, in denen das Oligosid bevorzugterweise Lactose, Melibiose oder Stachiose ist:

. (III) Oligosid 1-NH-CO-R, worin
R ein Alkylrest mit 2 bis 18 Kohlenstoffatomen ist, der 0, 1 oder 2 Doppelbindungen enthält,
. (IV) Oligosid 1-NH-CO-R-CO-NH-1-Oligosid, worin
R = $(CH_2)_n$ ist und n zwischen 2 und 8 enthalten ist,

c) - einem Alkylamin, das durch eine Aldonsäure acyliert ist, die durch Oxidation eines Oligosids erhalten ist,

. (V) Oligosid-CO-NH-R, worin
R die gleiche Bedeutung hat wie in Formel (III),
. (VI) Oligosid CO-NH-R-NH-CO-Oligosid, wo R die gleiche Bedeutung wie in Formel (III) hat,

d) - oder ein Reduktionsprodukt von Schiffschen Basen, das von Oligosiden mit aliphatischen Mono- oder Diaminen gebildet ist und eine der folgenden Formeln aufweist:

. (VII) Gal-$(Hex)_n$-X-HN-R,
. (VIII) Gal-$(Hex)_n$-X-HN-R-NH-X-$(Hex)_n$-Gal,
worin:

Hex ein Hexose- (oder Pentose-) Rest ist,
n = 0, 1 oder 2,
X = ein Hexitol (oder Pentitol-) Rest ist, und
R die gleiche Bedeutung wie in (III) hat.

**11.** Dermo-kosmetische Zusammensetzung, dadurch gekennzeichnet, daß sie Melibiose enthält.

**12.** Verfahren zum Herstellen einer Zusammensetzung nach einem der Ansprüche 8 bis 10, dadurch gekennzeichnet, daß die Oligosaccharidderivate mittels chemischer Synthese erhalten werden.

**13.** Verfahren zum Herstellen einer Zusammensetzung nach einem der Ansprüche 8 bis 11, dadurch gekennzeichnet, daß es die folgenden Schritte umfaßt:

a) Extraktion von Zuckern ausgehend von einem biologischen Medium, insbesondere Milch, Blut oder Plazenta,
b) enzymatischer Abbau der extrahierten Zucker oder chemische Hydrolyse,
c) Abtrennen der gesuchten Oligosaccharide.

**14.** Verfahren zum Herstellen einer Zusammensetzung nach einem der Ansprüche 8 bis 11, bestimmt für die Prävention oder Behandlung von Alterungseffekten, dadurch gekennzeichnet, daß es den Einbau eines Oligosaccharides, das 2 bis 5 Zuckerreste enthält, die eine Galactose an einem nicht-reduzierenden Ende umfassen, oder eines Derivates eines solchen Oligosaccharides in ein für lokale Verabreichung akzeptables Vehikel umfaßt, welches

0,5 % bis 6 %, bevorzugterweise 1 bis 4 % Gewicht pro Volumen der Zusammensetzung darstellt.

MELIBIOSE

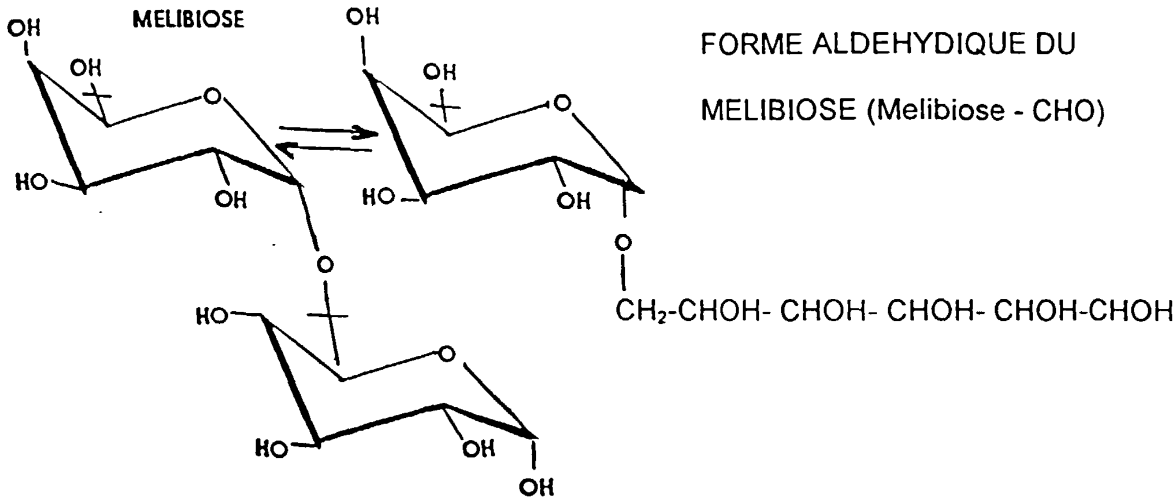

FORME ALDEHYDIQUE DU

MELIBIOSE (Melibiose - CHO)

Melibiose CHO + $H_2N$ - $(CH_2)_6$ -$NH_2$ + OHC - Melibiose

↓ ↖ 1,6 diaminohexane

Melibiose - CH = N - $(CH_2)_6$ - N = CH - Melibiose

(base de Schiff instable)

$NaCNBH_4$ ↓

[Melibiose - $CH_2$]- NH - $(CH_2)_6$ - NH - $CH_2$ - Melibiose

MELIBIONITOL

Melibionitol - NH ( $CH_2)_6$ - NH - Melibionitol

di - melibionityl - diaminohexane

FIGURE 1